# EUROPÄISCHE PATENTANMELDUNG

(11) **EP 1 902 632 A1**
(43) Veröffentlichungstag der Anmeldung: **26.03.2008**
(21) Anmeldenummer: 07116582.3
(22) Anmeldetag: 17.09.2007
(51) Int. Cl.: A23L 1/226, C07C 69/587, C11B 9/00

(54) **Dekonjugierte Alkadiensäureester als Riech- und/oder Aromastoffe**

(30) Priorität: 22.09.2006 DE 102006044849
(71) Anmelder: Symrise GmbH & Co. KG, 37603 Holzminden (DE)
(72) Erfinder: Ley, Jakob, 37603, Holzminden (DE); Riess, Thomas, 37603, Holzminden (DE)
(74) Vertreter: Stilkenböhmer, Uwe Michael

(57) **Zusammenfassung**

Beschrieben wird die Verwendung
(i) eines Fettsäureesters der Formel (I) oder
(ii) einer Mischung von zwei oder mehr unterschiedlichen Fettsäureestern der Formel (I)

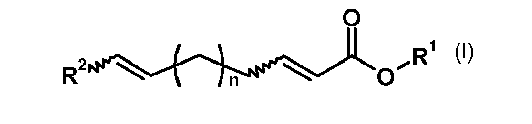

wobei jeweils
R¹
einen Alkylrest mit 1 bis 10 C-Atomen,
einen Cycloalkylrest mit 3 bis 8 C-Atomen,
einen Alkenylrest mit 2 bis 10 C-Atomen oder
einen Cycloalkenylrest mit 3 bis 8 C-Atomen
darstellt,
R²
einen Alkylrest mit 1 bis 10 C-Atomen darstellt,
n
1, 2, 3, 4 oder 5 bedeutet,
und
die beiden Doppelbindungen im Fettsäurerest unabhängig voneinander (E) oder (Z) konfiguriert sind,
als Riech- und/oder Aromastoff.

## Beschreibung

Die Erfindung betrifft die Verwendung bestimmter dekonjugierter, im Fettsäurerest zweifach ungesättigter, Fettsäureester mittlerer Kettenlänge (Alkadiensäureester) als Riech- und/oder Aromastoffe sowie Zubereitungen, insbesondere Riech- und Aromakompositionen, kosmetische, der Körperpflege dienende, haushaltstechnische, der Ernährung, dem Genuss oder der Mundhygiene dienende sowie dermatologische oder orale pharmazeutische Zubereitungen, en t-haltend diese Alkadiensäureester.

Der sogenannte Birnenester (2E,4Z)-Decadiensäureethylester und seine geometrischen Isomere sind als Riech- und Aromastoffe schon länger bekannt (vgl. US 5,753,473; Chimia 2001, 55, 397-400). Der Geruchs- und Geschmackseindruck ist zwar charakteristisch (fruchtig, Williams-Birne), dabei insgesamt jedoch relativ schwach. Zudem sind die Verbindungen aufgrund ihres vollständig konjugierten Doppelbindungssystems sehr empfindlich gegen Licht- und Säureeinfluss (z.B. Isomerisierung der Doppelbindungen) und können sehr leicht oxidieren, wobei sowohl Abbau- als auch Polymerisierungsprodukte entstehen, die den (sensorischen) Wert der Zubereitungen erniedrigen.

Die für fruchtige, insbesonders wässrig-fruchtige, riechende und/oder schmeckende Zubereitungen gerne verwendeten Alkadienale, z.B. (2E,7Z)-Nonadienal, sind aus anwendungstechnischer Sicht oft nicht ausreichend stabil. Zudem besteht bei ungesättigten Aldehyden die Gefahr, ein reizendes und/oder sensibilisierendes Potential an der Haut zu zeigen, was die Verwendbarkeit stark einschränkt oder sogar verhindert.

Daher besteht weiterhin Bedarf nach wässrig-fruchtig riechenden und/oder schmeckenden Verbindungen, die stabiler sind als die genannten Aldehyde oder vollständig konjugierte Alkadiensäureester, und sich zudem einfach in hohen Ausbeuten herstellen lassen.

Die gestellte Aufgabe wird erfindungsgemäß gelöst durch die Verwendung
(i) eines Fettsäureesters der Formel (I) oder
(ii) einer Mischung von zwei oder mehr unterschiedlichen Fettsäureestern der Formel (I) 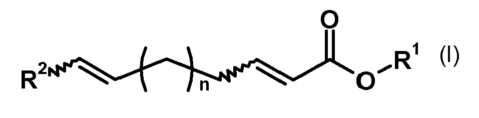
wobei jeweils
- R¹: einen Alkylrest mit 1 bis 10 C-Atomen,
einen Cycloalkylrest mit 3 bis 8 C-Atomen,
einen Alkenylrest mit 2 bis 10 C-Atomen oder
einen Cycloalkenylrest mit 3 bis 8 C-Atomen
darstellt,
- R²: einen Alkylrest mit 1 bis 10 C-Atomen darstellt,
- n: 1, 2, 3, 4 oder 5 bedeutet,
und
die beiden Doppelbindungen im Fettsäurerest unabhängig voneinander (E) oder (Z) konfiguriert sind,
als Riech- und/oder Aromastoff.

Erfindungsgemäß bevorzugt ist eine Verwendung der vorstehend genannten Art, wobei der Fettsäureester der Formel (I) bzw. zumindest ein oder jeder Fettsäureester der Formel (I) in der Mischung
(i) höchstens 18 C-Atome und mindestens 9 C-Atome umfasst,
(ii) höchstens 16 C-Atome und mindestens 9 C-Atome umfasst
   und/oder
(iii) höchstens 16 C-Atome und mindestens 10 C-Atome umfasst.

Alkylreste im Sinne der Erfindung sind lineare oder verzweigte, gesättigte Kohlenwasserstoffreste mit 1 bis 10, bevorzugt 1 bis 4 C-Atomen, vorzugsweise Methyl, Ethyl, 1-Propyl, 2-Propyl, 1-Butyl, 2-Butyl, tert-Butyl.

Cycloalkylreste im Sinne der Erfindung sind cyclische, gesättigte Kohlenwasserstoffreste mit 3 bis 8 C-Atomen, vorzugsweise Cyclopropyl, Cyclobutyl, Cyclopentyl, Cyclohexyl.

Alkenylreste im Sinne der Erfindung sind lineare oder verzweigte, einfach ungesättigte Kohlenwasserstoffreste mit 2 bis 10, bevorzugt 2 bis 4 Kohlenstoffatomen, vorzugsweise Ethenyl, 1-Propenyl, 2-Propenyl, 1-Buten-1-yl, 1-Buten-2-yl, 2-Buten-2-yl, 2-Buten-1-yl, 3-Buten-1-yl und 2-Methyl-2-propen-1-yl.

Cycloalkenylreste im Sinne der Erfindung sind cyclische, einfach ungesättigte Kohlenwasserstoffreste mit 3 bis 8, bevorzugt 3 bis 6 Kohlenstoffatomen, vorzugsweise 1-Cyclopropenyl, 2-Cyclopropenyl, 1-Cyclobutenyl, 2-Cyclobutenyl, 1-Cyclopentenyl, 2-Cyclopentenyl.

Bevorzugt zur Verwendung als Riech- und/oder Aromastoff sind Fettsäureester bzw. Mischungen von zwei oder mehr unterschiedlichen Fettsäureestern der Formel (I), wobei für den, zumindest einen oder jeden Fettsäureester der Formel (I)
- R¹: einen Alkylrest mit 1 bis 4 C-Atomen,
einen Cycloalkylrest mit 3 bis 6 C-Atomen,
einen Alkenylrest mit 2 bis 4 C-Atomen oder
einen Cycloalkenylrest mit 3 bis 6 C-Atomen
darstellt,
und/oder (vorzugsweise und)
- R²: einen Alkylrest mit 1 bis 3 C-Atomen darstellt
und/oder (vorzugsweise und)
- n: 2, 3 oder 4 bedeutet.

Dabei gelten die vorstehenden Angaben zu R¹, R² und n vorzugsweise zugleich.

Ganz besonders bevorzugt sind Fettsäureester, wobei
- R¹: einen Alkylrest bedeutet, der ausgewählt ist aus der Gruppe bestehend aus Methyl, Ethyl, 1-Propyl, 2-Propyl, 1-Butyl, 2-Butyl und tert-Butyl,
einen Cycloalkylrest bedeutet, der ausgewählt ist aus der Gruppe bestehend aus Cyclopropyl, Cyclobutyl, Cyclopentyl und Cyclohexyl,
einen Alkenylrest bedeutet, der ausgewählt ist aus der Gruppe bestehend aus Ethenyl, 1-Propenyl, 2-Propenyl, 1-Buten-1-yl, 1-Buten-2-yl, 2-Buten-2-yl, 2-Buten-1-yl, 3-Buten-1-yl und 2-Methyl-2-propen-1-yl
oder
einen Cycloalkenylrest bedeutet, der ausgewählt ist aus der Gruppe bestehend aus1-Cyclopropenyl, 2-Cyclopropenyl, 1-Cyclobutenyl, 2-Cyclobutenyl, 1-Cyclopentenyl und 2-Cyclopentenyl
und/oder (vorzugsweise und)
- R²: einen Alkylrest bedeutet, der ausgewählt ist aus der Gruppe bestehend aus Methyl, Ethyl und 1-Propyl.

Wiederum liegen vorzugsweise die besonders bevorzugten Bedeutungen von R¹ und R² zugleich vor.

Jeweils gilt, dass die Doppelbindungen im Fettsäurerest eines Alkadiensäureesters der Formel (I) unabhängig voneinander (E) oder (Z) konfiguriert sind.

Besonders bevorzugt ist die Verwendung (i) eines Fettsäureesters der Formel (I) oder (ii) einer Mischung von zwei oder mehr unterschiedlichen Fettsäureestern der obigen Formel (I), wobei der, zumindest ein oder jeder Fettsäureester der Formel (I) ausgewählt ist aus der Gruppe bestehend aus:
2,7-Nonadiensäuremethylester,
2,7-Decadiensäuremethylester.
2,7-Undecadiensäuremethylester,
2,8-Decadiensäuremethylester,
2,8-Undecadiensäuremethylester,
2,8-Dodecadiensäuremethylester,
2,9-Undecadiensäuremethylester,
2,9-Dodecadiensäuremethylester,
2,9-Tridecadiensäuremethylester,
2,7-Nonadiensäureethylester,
2,7-Decadiensäureethylester,
2,7-Undecadiensäureethylester,
2,8-Decadiensäureethylester,
2,8-Undecadiensäureethylester,
2,8-Dodecadiensäureethylester,
2,9-Undecadiensäureethylester,
2,9-Dodecadiensäureethylester,
2,9-Tridecadiensäureethylester,
2,7-Nonadiensäureallylester,
2,7-Decadiensäureallylester,
2,7-Undecadiensäureallylester,
2,8-Decadiensäureallylester,
2,8-Undecadiensäureallylester,
2,8-Dodecadiensäureallylester,
2,9-Undecadiensäureallylester,
2,9-Dodecadiensäureallylester,
2,9-Tridecadiensäureallylester,
2,7-Nonadiensäure-tert.-butylester,
2,7-Decadiensäure-tert.-butylester,
2,7-Undecadiensäure-tert.-butylester,
2,8-Decadiensäure-tert.-butylester,
2,8-Undecadiensäure-tert.-butylester,
2,8-Dodecadiensäure-tert.-butylester,
2,9-Undecadiensäure-tert.-butylester
2,9-Dodecadiensäure-tert.-butylester und
2,9-Tridecadiensäure-tert.-butylester.

Vergleiche insoweit auch die beigefügte Tabelle 1.

Besonders bevorzugte Alkadiensäureester der Formel (I) sind 2,7-Decadiensäureester (R² = Ethyl; n = 2) und 2,8-Undecadiensäureester (R² = Ethyl; n = 3) und deren Mischungen, wobei weiter bevorzugt R¹ einen Alkylrest mit 1 bis 4 C-Atomen oder einen Alkenylrest mit 2 bis 4 C-Atomen darstellt.

In besonders bevorzugten Verwendungen ist der, zumindest ein oder jeder Fettsäureester der Formel (I) ausgewählt aus der Gruppe bestehend aus:
2,7-Decadiensäureethylester,
2,7-Decadiensäuremethylester,
2,7-Decadiensäure-tert.-butylester,
2,7-Decadiensäureallylester,
2,8-Undecadiensäureethylester,
2,8-Undecadiensäuremethylester,
2,8-Undecadiensäure-tert.-butylester und
2,8-Undecadiensäureallylester.

Aufgrund ihrer Geruchscharakteristik ist es vorteilhaft, wenn der, zumindest ein oder jeder Fettsäureester der Formel (I) ausgewählt ist aus der Gruppe bestehend:
2,7-Decadiensäureethylester,
2,7-Decadiensäuremethylester,
2,8-Undecadiensäureethylester und
2,8-Undecadiensäuremethylester.

Dabei wiederum bevorzugt sind jeweils die (2E,7Z)- bzw. (2E,8Z)-Isomere der genannten 2,7-Decadiensäureester und 2,8-Undecadiensäureester bzw. deren Mischungen, in denen die jeweiligen (2E,7Z)- bzw. (2E,8Z)-lsomere zumindest 80 mol.-%, weiter bevorzugt zumindest 90 mol.-%, bezogen auf die Gesamtmenge an eingesetzten Alkadiensäureester der Formel (I) ausmachen.

Überraschend und für den Fachmann nicht vorauszusehen war der sehr intensive und interessante Geruch der Alkadiensäurester der Formel (I), der nicht mehr birnenartig ist, sondern grün, fruchtig, wässrig, blumig und mit Aspekten von Melone, Gurke, Apfel, Veilchen und/oder Maiglöckchen, zum Teil aldehydig und etwas fettig. Ferner zeigen die Alkadiensäurester der Formel (I) eine hohe Haftfähigkeit und eignen sich daher besonders gut für den Einsatz in Parfümölen und/oder Aromenkompositionen.

Es wurde nun im Rahmen der vorliegenden Erfindung gefunden, dass sich die genannten (insbesondere die als (besonders) bevorzugt bezeichneten) Alkadiensäurester der Formel (I) hervorragend zum Vermitteln, Modifizieren und/oder Verstärken eines Geruchs mit einer oder mehreren der Noten grün, fruchtig, wässrig, blumig, aldehydig, fettig, Melone, Gurke, Apfel, Veilchen und/oder Maiglöckchen eignen.

Insbesondere sind die genannten (insbesondere die oben als (besonders) bevorzugt bezeichneten) Alkadiensäurester der Formel (I) zur Einarbeitung in Parfümöle geeignet, speziell zur Einarbeitung in solche, welche grüne, fruchtige, blumige, aldehydige und/oder wässrige Noten aufweisen. Dabei können die Alkadiensäurester der Formel (I) in vielfältigen Parfümkompositionen eingesetzt werden, z.B. auch in Blumenduft-Themen (siehe auch Beispiel 5).

Es wurde zudem gefunden, dass sich die genannten Alkadiensäureester der Formel (I) (insbesondere die oben als (besonders) bevorzugt bezeichneten) hervorragend zum Vermitteln, Modifizieren und/oder Verstärken eines Geschmacks mit einer oder mehreren der Noten saftig, grün, Gurke, schalig, Melone, Weintraube und/oder Aldehyd eignen.

Insbesondere sind die Alkadiensäureester der Formel (I) (insbesondere die oben als (besonders) bevorzugt bezeichneten) zur Einarbeitung in Aromenkompositionen geeignet, welche grüne, fruchtige, saftige und/oder wässrige Noten aufweisen, insbesondere Noten von (Wasser)Melone bzw. Gurke.

Es wurde ferner gemäß einem weiteren Aspekt der Erfindung gefunden, dass sich die Verbindungen der Formel (I), besonders die oben als bevorzugt bezeichneten, insbesondere aber 2,8-Undecadiensäureester der Formel (I), und speziell deren (2E,8Z)-Isomere, hervorragend zum Verstärken eines Geruchs- oder Geschmacks eignen, d.h. dass die Verbindungen der Formel (I) als sogenannte Booster (Verstärker, Enhancer) fungieren können. Daher betrifft die Erfindung auch die Verwendung von Verbindungen der Formel (I) als Booster (Verstärker; Enhancer) für andere Riech- oder Aromastoffe, insbesondere für andere Riech- oder Aromastoffe mit grünen, frischen, blumigen, fruchtigen und/oder aldehydigen Noten.

Die vorliegende Erfindung betrifft auch parfümierte oder aromatisierte Artikel (insbesondere Riech- oder Aromastoffkompositionen und Artikel, welche eine Riech- oder Aromastoffkomposition umfassen), Verfahren zum Vermitteln, Modifizieren und/oder Verstärken bestimmter Geruchs- und/oder Geschmacksnoten sowie neue Verbindungen der Formel (I).

Unter den Alkadiensäurestern der Formel (I) sind lediglich einzelne bereits bekannt, die somit - für sich betrachtet - nicht Gegenstand der vorliegenden Erfindung sind.

Die 2,8-Undecadiensäureester der Formel (I) sind neu.

In Helv. Chim. Acta, 1978, 885-898 werden auf Seite 895 die Verbindungen (2E, 7E)- und (2E, 7Z)-Decadiensäuremethylester sowie die Verbindung (2E, 7Z)-Decadiensäureethylester offenbart. Insbesondere wird ausgeführt, dass Triethylphosphonoessigsäureethyl- und -methylester 10 mmol in 5 ml Benzol mit 10 mmol NaH-Dispersion (von Mineralöl frei gewaschen) bei Raumtemperatur während einer Stunde umgesetzt wurden. Zur klaren Ylidlösung wurden 10 mmol 5-Octenal in 5 ml Benzol tropfenweise bei Raumtemperatur gegeben. Es bildete sich ein zäher Niederschlag. Daraufhin wurde 5 Stunden bei 60-70° weiter gerührt, gekühlt, der Niederschlag abfiltriert, mit warmem Benzol ausgewaschen, die Filtrate wurden eingedampft und der Rückstand im Kugelrohr destilliert. Hieraus ergab sich (2E, 7E)-Decadiensäuremethylester bzw. (2E, 7Z)-Decadiensäureethylester.

Ferner wird auf der besagten Seite des genannten Dokumentes offenbart, dass der Ethylester mit 2 N HCI in Methanol bei Raumtemperatur während 40 Stunden umgeestert wurde, so dass (2E, 7Z)-Decadiensäure-methylester resultierte.

Zudem wird auf der besagten Seite des genannten Dokumentes offenbart, dass sich die Methylester unter bestimmten Bedingungen trennen ließen, wobei insbesondere vom Einspritzen einer ätherischen Lösung die Rede ist.

Schließlich offenbart das genannte Dokument auf der besagten Seite auch noch, dass 1 mmol des Decadiensäure-methylesters in 5 ml Benzol mit 1 mmol m-Chlorperbenzoesäure umgesetzt wurde.

Aus den vorstehenden Angaben zum Offenbarungsgehalt der genannten Veröffentlichung und aus einem Studium der Veröffentlichung selbst ergibt sich, dass (2E, 7E)- und (2E, 7Z)-Decadiensäuremethylester sowie (2E, 7Z)-Decadiensäureethylester im Rahmen der Veröffentlichung lediglich als Synthesezwischenprodukte eingesetzt wurden. Die erfindungsgemäße Verwendung der 2,7-Decadiensäuremethyl- und ethylester als Riech- und Aromastoff ist in Helv. Chim. Acta, 1978, 885-898 nicht offenbart.

Ingesamt ist die Verwendung von 2,7-Decadiensäureestern der Formel (I) als Riech- oder Aromastoff oder deren entsprechende Verwendung in Artikeln aus dem Stand der Technik nicht bekannt.

Die Alkadiensäureester der Formel (I) können hergestellt werden, indem ein Aldehyd der Formel (II) 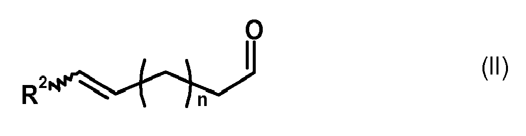 wobei R² und n die obige Bedeutung (vorzugsweise eine oben als bevorzugt genannte Bedeutung) haben
mit
einem Phosphonoester der allgemeinen Formel (III) 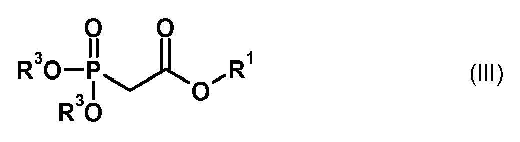 wobei R¹ die obige Bedeutung (vorzugsweise eine oben als bevorzugt genannte Bedeutung) hat,
und jedes R³ einen linearen oder verzweigten Alkylrest darstellt, vorzugsweise einen Alkylrest mit 1 bis 10, bevorzugt 1 bis 4 C-Atomen, vorzugsweise Methyl, Ethyl, 1-Propyl, 2-Propyl, 1-Butyl, 2-Butyl, tert-Butyl,
umgesetzt wird. Die Reaktionsmischung wird üblicherweise aufgearbeitet, und gegebenenfalls werden die entstandenen Alkadiensäureester der Formel (I) aufgereinigt.

Die Umsetzung erfolgt dabei regelmäßig in Gegenwart einer Hilfsbase und eines Lösungsmittels oder eines Lösungsmittelgemisches, wobei sowohl wässrige als auch nichtwässrige Systeme, bevorzugt unter Zuhilfenahme eines Phasentransferkatalysators verwendet werden können.

Besonders bevorzugt wird die Umsetzung in Gegenwart von Wasser durchgeführt, wobei eine schwache Base, bevorzugt Kaliumhydrogencarbonat oder Kaliumcarbonat, und ein Phasentransferkatalysator, bevorzugt ein quartäres Ammoniumsalz, z.B. Tetrabutylammoniumbromid oder Cetyltrimethylammoniumchlorid, verwendet wird und die Reaktion bei Temperaturen von 0 bis 100 °C, bevorzugt 10 bis 80°C durchgeführt wird.

Zur Aufarbeitung und Reinigung wird in einer besonders bevorzugten Auführungsform die Mischung nach Beendigung der Reaktion, ggfs. nach Zugabe eines lipophilen Lösungsmittels, bevorzugt eines Kohlenwasserstoffs, einfach in lipophile und wässrige Phase getrennt, die lipophile Mischung mit Wasser gewaschen und sodann unter Vakuum schonend fraktioniert destilliert, wobei man hohe Ausbeuten des bevorzugten Hauptisomers in Riechstoffqualität erhält.

Wie erwähnt betrifft die vorliegende Erfindung auch parfümierte oder aromatisierte Artikel, und zwar einschließlich Zubereitungen, Halbfertigwaren sowie Riech-, Aroma- und Geschmackstoffkompositionen.

Erfindungsgemäße parfümierte oder aromatisierte Artikel umfassen:
(a)
   (i) einen Fettsäureester der Formel (I) oder
   (ii) eine Mischung von zwei oder mehr unterschiedlichen Fettsäureestern der Formel (I) 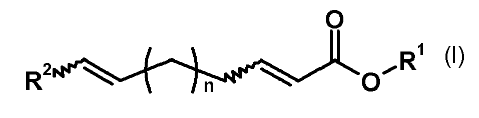 wobei jeweils
      - R¹: einen Alkylrest mit 1 bis 10 C-Atomen,
      einen Cycloalkylrest mit 3 bis 8 C-Atomen,
      einen Alkenylrest mit 2 bis 10 C-Atomen oder
      einen Cycloalkenylrest mit 3 bis 8 C-Atomen
      darstellt,
      - R²: einen Alkylrest mit 1 bis 10 C-Atomen darstellt,
      - n: 1, 2, 3, 4 oder 5 bedeutet,
   und
   die beiden Doppelbindungen im Fettsäurerest unabhängig voneinander (E) oder (Z) konfiguriert sind,
   mit der Maßgabe, dass der Artikel
   (i) kein (2E,7E)- oder (2E,7Z)-Decadiensäuremethylester oder (2E,7Z)-Decadiensäureethylester sowie weitere Bestandteile umfassender Artikel (insbesondere Zusammensetzung) ist, wie er in Helv. Chim. Acta, 1978, 885-898 auf Seite 895 (insbesondere in den oben gewürdigten Textpassagen) beschrieben ist
      und/oder
   (ii) kein Benzol umfasst
      und/oder
   (iii) kein 5-Octenal umfasst
      und/oder
   (iv) kein Methanol umfasst
   sowie
(b) einen, zwei, drei oder mehr weitere Riech- oder Aromastoffe.

Erfindungsgemäß bevorzugte parfümierte oder aromatisierte Artikel (einschließlich Zubereitungen) sind insbesondere kosmetische, der Körperpflege dienende, haushaltstechnische, der Ernährung, dem Genuss oder der Mundhygiene (einschließlich Mundpflege) dienende sowie dermatologische oder (orale) pharmazeutische Zubereitungen.

Es ist vorteilhaft, einen oder mehrere Alkadiensäureester der Formel (I) mit zumindest einem weiteren Riech- oder Aromastoff zu kombinieren und so eine neue Riech- oder Aromastoffkomposition (als Beispiel eines erfindungsgemäßen Artikels) zu bilden. Auf diese Weise lassen sich besonders interessante und natürliche neue und originelle Duftnoten kreieren. Riechstoffe, die zur Kombination vorteilhafterweise geeignet sind, finden sich z.B. in S. Arctander, Perfume and Flavor Materials, Vol. I und II, Montclair, N. J. 1969, Eigenverlag, oder K. Bauer et al., Common Fragrance and Flavor Materials, 4th Edition, Wiley-VCH, Weinheim 2001. Im einzelnen seien genannt:
Extrakte aus natürlichen Rohstoffen wie Etherische Öle, Concretes, Absolues, Resine, Resinoide, Balsame, Tinkturen wie z. B.

Ambratinktur; Amyrisöl; Angelicasamenöl; Angelicawurzelöl; Anisöl; Baldrianöl; Basilikumöl; Baummoos -Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl ; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl ; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierten Inhaltsstoffen;

Einzel-Riechstoffe aus der Gruppe der Kohlenwasserstoffe, wie z.B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole wie z.B. Hexanol; Octanol; 3-Octanol; 2,6-Dimethylheptanol; 2-Methyl-2-heptanol; 2-Methyl-2-octanol; (E)-2-Hexenol; (E)-und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale wie z.B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(E/Z)-3-hexen; der aliphatischen Ketone und deren Oxime wie z.B. 2-Heptanon; 2-Octanon; 3-Octanon; 2-Nonanon; 5-Methyl-3-heptanon ; 5-Methyl-3-heptanonoxim; 2,4,4,7-Tetramethyl-6-octen-3-on; 6-Methyl-5-hepten-2-on;
der aliphatischen schwefelhaltigen Verbindungen wie z.B. 3-Methylthio-hexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B. 2-Nonensäurenitril; 2-Undecensäurenitril; 2-Tridecensäurenitril; 3,12-Tridecadiensäurenitril; 3,7-Dimethyl-2,6-octadiensäurenitril; 3,7-Dimethyl-6-octensäurenitril;
der Ester von aliphatischen Carbonsäuren wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat, ; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4- decadienoat; Methyl-2-octinat; Methyl-2-noninat; Allyl-2-isoamyloxyacetat; Methyl-3,7-dimethyl-2,6-octadienoat;4-Methyl-2-pentyl-crotonat;
der acyclischen Terpenalkohole wie z. B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, lsovalerianate, Pentanoate, Hexanoate, Crotonate, Tiglinate und 3-Methyl-2-butenoate;
der acyclischen Terpenaldehyde und -ketone wie z. B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole wie z. B. Menthol; lsopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate;
der cyclischen Terpenaldehyde und -ketone wie z. B. Menthon; Isomenthon ; 8-Mercaptomenthan-3-on ; Carvon; Campher; Fenchon; alpha-lonon; beta-lonon; alpha-n-Methylionon; beta-n-Methylionon; alpha-lsomethylionon; betalsomethylionon; alpha-lron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on;2-Methyl-4-(2,6,6-trimethyl-1-cyclohexen-1-yl)-2-butenal; Nootkaton ; Dihydronootkaton ; 4,6,8-Megastigmatrien-3-on; alpha-Sinensal ; beta-Sinensal ; acetyliertes Cedernholzöl (Methylcedrylketon);
der cyclischen Alkohole wie z.B. 4-tert.-Butylcyclohexanol ; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z.B. alpha,3,3-Trimethylcyclohexylmethanol;1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. Cineol; Cedrylmethylether; Cyclododecylmethylether;1,1-Dimethoxycyclododecan; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone wie z.B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon;
der cycloaliphatischen Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd; der Ester cyclischer Alkohole wie z.B. 2-tert-Butylcyclohexylacetat; 4-tert-Butylcyclohexylacetat; 2-tert-Pentylcyclohexylacetat; 4-tert-Pentylcyclohexylacetat; 3,3,5-Trimethylcyclohexylacetat; Decahydro-2-naphthylacetat;2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Alkohole wie z.B.1-Cyclohexylethylcrotonat;
der Ester cycloaliphatischer Carbonsäuren wie z. B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der araliphatischen Alkohole wie z.B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal;
Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 2-Methyl-3-(4-isobutylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon;2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1 H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3- cyclohexencarbald e-hyd; 6-Isopropylchinolin; 6-Isobutylchinolin; 6-sec.-Butylchinolin;2-(3-Phenylpropyl)pyridin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid;4-Methyl-1,4-decanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

In erfindungsgemäßen Riech- oder Aromastoffkompositionen beträgt die eingesetzte Gesamtmenge der einen oder mehreren Alkadiensäureester der Formel (I) vorzugsweise 0,00001 bis 90 Gew.-%, vorzugsweise 0,001 bis 70 Gew.-% und besonders bevorzugt 0,01 bis 50 Gew.-%, bezogen auf die Gesamtmenge der Riech- oder Aromastoffkomposition.

In erfindungsgemäßen Riech- oder Aromastoffkompositionen liegt das Gewichtsverhältnis der Gesamtmenge an Fettsäurestern der Formel (I) zu der Gesamtmenge an weiteren Riech- oder Aromastoffen im Bereich von 1 : 1000 bis 1 : 0,5.Erfindungsgemäße Riech- oder Aromastoffkompositionen, die einen oder mehrere Alkadiensäureester der Formel (I) enthalten, können in flüssiger Form, unverdünnt oder mit einem Lösungsmittel verdünnt zur Parfümierung oder Aromatisierung eingesetzt werden. Geeignete Lösungsmittel hierfür sind z.B. Ethanol, Isopropanol, Diethylenglycolmonoethyleter, Glycerin, Propylenglycol, 1,2-Butylenglycol, Dipropylenglycol, Diethylphtalat, Triethylcitrat, Isopropylmyristat, Triacetin usw.

Benzol und Methanol sind regelmäßig jeweils kein Bestandteil einer erfindungsgemäßen Riech- oder Aromastoffkomposition.

Des weiteren können erfindungsgemäße Riech- oder Aromastoffkompositionen, die einen oder mehrere Alkadiensäureester der Formel (I) enthalten, an einem Trägerstoff adsorbiert sein, der sowohl für eine feine Verteilung der Riech- oder Aromastoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer, Cellulose-basierende Stoffe, Zucker, Dextrine (z.B. Maltodextrin) oder Kunststoffe wie PVC, Polyvinylacetate oder Polyurethane sein. Die Kombination aus erfindungsgemäßer Komposition und Trägerstoff stellt einen beispielhaften erfindungsgemäßen Artikel dar.

Erfindungsgemäßen Riech- oder Aromastoffkompositionen, die einen oder mehrere Alkadiensäureester der Formel (I) enthalten, können auch mikroverkapselt, sprühgetrocknet, als Einschlusskomplexe oder als Extrusions-Produkte (d. h. erfindungsgemäße Artikel) vorliegen und in dieser Form z. B. einem zu parfümierenden oder aromatisierenden Produkt hinzugefügt werden.

Ein bevorzugter erfindungsgemäßer parfümierter oder aromatisierter Artikel umfasst eine Gesamtmenge an Fettsäureestern der Formel (I) die ausreicht, eine
- Geruchsnote des Typs grün, fruchtig, wässrig, blumig, aldehydig, fettig, Melone, Gurke, Apfel, Veilchen und/oder Maiglöckchen
   oder
- Geschmacksnote des Typs saftig, grün, Gurke, schalig, Melone, Weintraube und/oder Aldehyd
zu vermitteln, zu modifizieren und/oder zu verstärken. Vorzugsweise handelt es sich bei einem solchen erfindungsgemäßen Artikel um eine Riech- oder Aromastoffkomposition.

Gegebenenfalls können die Eigenschaften der derart modifizierten Kompositionen durch sog. "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden. Die resultierenden Produkte stellen wiederum erfindungsgemäße Artikel dar.

Die Mikroverkapselung der erfindungsgemäßen Riech- oder Aromastoffkompositionen zu erfindungsgemäßen Artikeln kann beispielsweise durch das sogenannte Koazervationsverfahren mit Hilfe von Kapselmaterialien z.B. aus polyurethanartigen Stoffen oder Weichgelatine, erfolgen. Die sprühgetrockneten Riech - oder Aromastoffkompositionen können beispielsweise durch Sprühtrocknung einer die Riech- oder Aromastoffkomposition enthaltenden Emulsion, bzw. Dispersion hergestellt werden, wobei als Trägerstoffe modifizierte Stärken, Proteine, Dextrin und pflanzliche Gummen verwendet werden können. EinschlussKomplexe können z.B. durch Eintragen von Dispersionen von der Riech- oder Aromastoffkomposition und Cyclodextrinen oder Harnstoffderivaten in ein geeignetes Lösungsmittel, z.B. Wasser, hergestellt werden. Extrusions-Produkte können durch Verschmelzen der Riech- oder Aromastoffkompositionen mit einem geeigneten wachsartigen Stoff und durch Extrusion mit nachfolgender Erstarrung, ggf. in einem geeigneten Lösungsmittel, z.B. Isopropanol, erhalten werden.

Die erfindungsgemäß zu verwendenden Alkadiensäureester der Formel (I) und Riechstoffkompositionen, die einen oder mehrere solcher Alkadiensäureester der Formel (I) enthalten, können in konzentrierter Form, in Lösungen oder in oben beschriebener modifizierter Form verwendet werden für die Herstellung von erfindungsgemäßen parfümierten Artikeln wie z.B. Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, Textilerfrischern, Bügelhilfen, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom ÖI-in-Wasser-, vom Wasser-in-ÖI- und vom Wasser-in-Öl-in-Wasser-Typ wie z.B. Hautcremes- und -lotionen, Gesichtscremes und -lotionen, Sonnenschutzcremes-und -lotionen, After-sun-cremes und - lotionen, Handcremes und -lotionen, Fußcremes und -lotionen, Enthaarungscremes und -lotionen, After-shave-Cremes und -lotionen, Bräunungscremes und - lotionen, Haarpflegeprodukten wie z.B. Haarsprays, Haargelen, festigenden Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien und Treibstoffen.

Die erfindungsgemäß zu verwendenden Alkadiensäureester der Formel (I) und deren Mischungen können in aromatisierte oder zu aromatisierende Artikel eingearbeitet werden, insbesondere der Ernährung, der Mundpflege oder dem Genuss dienende Zubereitungen.

Der Ernährung oder dem Genuss dienende Zubereitungen sind z.B. Backwaren (z.B. Brot, Trockenkekse, Kuchen, sonstiges Gebäck), Süßwaren (z.B. Schokoladen, Schokoladenriegelprodukte, sonstige Riegelprodukte, Fruchtgummi, Hart- und Weichkaramellen, Kaugummi), alkoholische oder nicht-alkoholische Getränke (z.B. Kaffee, Tee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (z.B. Frühstückscerealien, Müsliriegel, vorgegarte Fertigreis-Produkte), Milchprodukte (z.B. Milchgetränke, Milcheis, Joghurt, Kefir, Frischkäse, Weichkäse, Hartkäse, Trockenmilchpulver, Molke, Butter, Buttermilch, teilweise oder ganz hydrolisierte Milchprotein-haltige Produkte), Produkte aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte, Sojasoßen), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegarte Gemüse, in Essig eingelegte Gemüse, eingekochte Gemüse), Knabberartikel (z.B. gebackene oder frittierte Kartoffelchips oder Kartoffelteigprodukte, Brotteigprodukte, Extrudate auf Mais- oder Erdnussbasis), Produkte auf Fett- und Ölbasis oder Emulsionen derselben (z.B. Mayonnaise, Remoulade, Dressings, Würzzubereitungen), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden. Nach Einarbeiten der erfindungsgemäßzu verwendenden Alkadiensäureester der Formel (I) sind diese Zubereitungen erfindungsgemäße Zubereitungen.

Erfindungsgemäße Zubereitungen können z. B. als Halbfertigware oder als Würzmischung vorliegen.

Erfindungsgemäße Zubereitungen können insbesondere als Halbfertigware zur Herstellung weiterer der Ernährung oder dem Genuss dienenden Zubereitungen dienen, insbesondere in sprühgetrockneter Form. Erfindungsgemäße Zubereitungen können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen als Nahrungsergänzungsmittel vorliegen.

Der Mundpflege dienende erfindungsgemäße Zubereitungen sind insbesondere Mund- und/oder Zahnpflegemittel wie Zahnpasten, Zahngele, Zahnpulver, Zahnseide, Zahnstocher, Mundwässer und Kaugummis.

Weitere übliche Wirk-, Grund-, Hilfs- und Zusatzstoffe für der Ernährung, der Mundpflege oder dem Genuss dienende erfindungsgemäße Zubereitungen können in Mengen von 5 bis 99,99 Gew.-%, vorzugsweise 10 bis 80 Gew.-%, bezogen auf das Gesamtgewicht der Zubereitung, enthalten sein. Ferner können die Zubereitungen Wasser in einer Menge bis zu 99,99 Gew.-%, vorzugsweise 5 bis 80 Gew.-%, bezogen auf das Gesamtgewicht der Zubereitung, aufweisen.

Die erfindungsgemäßen Zubereitungen (als Beispiele erfindungsgemäßer Artikel), enthaltend einen oder mehrere Alkadiensäureester der Formel (I), werden gemäß einer bevorzugten Ausgestaltung hergestellt, indem der oder die Alkadiensäureester der Formel (I) als Substanz, als Lösung (z.B. in Ethanol, Wasser oder 1,2-Propylenglycol) oder in Form eines Gemisches mit einem festen oder flüssigen Trägerstoff (z.B. Maltodextrin, Stärke, Silicagel), sonstigen Aromen oder Aromastoffen und gegebenfalls weiteren Hilfsmitteln und/oder Stabilisatoren (z.B. natürliche oder künstliche Polysaccharide und/oder Pflanzengummen wie modifizierte Stärken oder Gummi Arabicum) in eine der Ernährung, der Mundpflege oder dem Genuss dienende Basis-Zubereitung eingearbeitet werden. Vorteilhafterweise können als Lösung und/oder Suspension oder Emulsion vorliegende erfindungsgemäße Zubereitungen auch durch Sprühtrocknung in eine feste erfindungsgemäße Zubereitung (Halbfertigware) überführt werden.

Die erfindungsgemäßen sprühgetrockneten festen Zubereitungen (als Beispiel erfindungsgemäßer Artikel) sind als Halbfertigwaren besonders gut zur Herstellung von weiteren erfindungsgemäßen Zubereitungen geeignet. In den erfindungsgemäßen sprühgetrockneten festen Zubereitungen sind vorzugsweise 50 bis 95 % Gew.-% Trägerstoffe, insbesondere Maltodextrin und/oder Stärke, 5 bis 40 % Hilfsstoffe, bevorzugt natürliche oder künstliche Polysaccharide und/oder Pflanzengummen wie modifizierte Stärken oder Gummi Arabicum enthalten.

Gemäß einer weiteren bevorzugten Ausführungsform werden zur Herstellung erfindungsgemäßer Zubereitungen der oder die Alkadiensäureester der Formel (I) und gegebenenfalls andere Bestandteile der erfindungsgemäßen Zubereitung zunächst in Emulsionen, in Liposomen, z.B. ausgehend von Phosphatidylcholin, in Microsphären, in Nanosphären oder auch in Kapseln, Granulaten oder Extrudaten aus einer für Lebens- und Genussmittel geeigneten Matrix, z.B. aus Stärke, Stärkederivaten (z.B. modifizierte Stärke), Cellulose oder Cellulosederivaten (z.B. Hydroxypropylcellulose), anderen Polysacchariden (z.B. Dextrin, Alginat, Curdlan, Carageenan, Chitin, Chitosan, Pullulan), natürlichen Fetten, natürlichen Wachsen (z.B. Bienenwachs, Carnaubawachs), aus Proteinen, z.B. Gelatineoder sonstigen Naturprodukten (z.B. Schellack) eingearbeitet. Dabei können je nach Matrix die Produkte durch Sprühtrocknung, Sprühgranulation, Schmelzgranulation, Koazervation, Koagulation, Extrusion, Schmelzextrusion, Emulsionsverfahren, Beschichtung (Coating) oder andere geeignete Verkapselungsverfahren und gegebenenfalls eine geeignete Kombination der vorgenannten Verfahren erhalten werden. In einem weiteren bevorzugten Herstellungsverfahren für eine erfindungsgemäße Zubereitung werden der oder die Alkadiensäureester der Formel (I) zunächst mit einem oder mehreren geeigneten Komplexbildnern, beispielsweise mit Cyclodextrinen oder Cyclodextrinderivaten, bevorzugt alpha- oder beta-Cyclodextrin, komplexiert und in dieser komplexierten Form eingesetzt.

Besonders bevorzugt ist eine erfindungsgemäße Zubereitung, bei der die Matrix so gewählt ist, dass die Alkadiensäureester der Formel (I) verzögert von der Matrix freigegeben werden, so dass eine langanhaltende Wirkung erzielt wird. Besonders bevorzugt ist insoweit eine Fett-, Wachs-, Polysaccharid- oder Proteinmatrix.

Als weitere Bestandteile für erfindungsgemäße, der Ernährung oder dem Genuss dienende Zubereitungen können übliche Grund-, Hilfs- und Zusatzstoffe für Nahrungs- oder Genussmittel verwendet werden, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose, Tagatose), Zuckeralkohole (z.B. Sorbit, Erythritol), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. γ-Aminobuttersäure, Taurin), Peptide (z.B. Glutahthion), native oder prozessierte Proteine (z.B. Gelatine), Enzyme (z.B. Peptidasen), Nukleinsäuren, Nucleotide, Geschmackskorrigenzien für unangenehme Geschmackseindrücke, weitere Geschmacksmodulatoren fürweitere, in der Regel nicht unangenehme Geschmackseindrücke, andere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole, Gummi arabicum), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), Bitterstoffe (z.B. Chinin, Coffein, Limonin, Amarogentin, Humolone, Lupolone, Catechine, Tannine), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Aromastoffe oder Riechstoffe sowie Geruchskorrigentien.

Erfindungsgemäße Zahnpflegemittel (als Basis für der Mundpflege dienende Zubereitungen), die einen oder mehrere Alkadiensäureester der Formel (I) enthalten, umfassen im Allgemeinen ein abrasives System (Schleif- oder Poliermittel), wie z.B. Kieselsäuren, Calciumcarbonate, Calciumphosphate, Alumiuniumoxide und/oder Hydroxylapatite, oberflächenaktive Substanzen wie z.B. Natriumlaurylsulfat, Natriumlaurylsarcosinat und/oder Cocamidopropylbetain, Feuchthaltemitteln wie z.B. Glycerin und/oder Sorbit, Verdickungsmittel, wie z.B. Carboxymethylcellulose, Polyethylenglycole, Carrageenan und/oder Laponite^{®}, Süßstoffe, wie z.B. Saccharin, Geschmackskorrigenzien für unangenehme Geschmackseindrücke, Geschmackskorrigenzien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Kühlwirkstoffen wie z.B. Menthol, Mentholderivate (z.B. L-Menthol, L-Menthyllactat, L-Menthylalkylcarbonate, Menthonketale, Menthancarbonsäureamide), 2,2,2-Trialkylessigsäureamiden (z.B. 2,2-Diisopropylpropionsäuremethylamid), Icilin und Icilin-Derivate, Stabilisatoren und aktive Wirkstoffe, wie z.B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromen und/oder Natriumbicarbonat oder Geruchskorrigentien.

Erfingungsgemäße Kaugummis (als weiteres Beispiel für der Mundpflege dienende Zubereitungen), welche einen oder mehrere Alkadiensäureester der Formel (I) enthalten, umfassen im allgemeinen eine Kaugummibase, d.h. eine beim Kauen plastisch werdende Kaumasse, Zucker verschiedener Arten, Zuckeraustauschstoffe, andere süß schmeckende Stoffe, Zuckeralkoholen, Geschmackskorrigenzien für unangenehme Geschmackseindrücke, andere Geschmacksmodulatoren für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Feuchthaltemittel, Verdicker, Emulgatoren, Aromen und Stabilisatoren oder Geruchskorrigentien.

Die vorliegende Erfindung betrifft auch ein entsprechendes Verfahren zum Vermitteln, Modifizieren und/oder Verstärken einer
- Geruchsnote des Typs grün, fruchtig, wässrig, blumig, aldehydig, fettig, Melone, Gurke, Apfel, Veilchen und/oder Maiglöckchen
   oder
- Geschmacksnote des Typs saftig, grün, Gurke, schalig, Melone, Weintraube und/oder Aldehyd,
wobei eine sensorisch wirksame Menge einer erfindungsgemäßen Riech- oder Aromastoffkomposition oder eines oder mehrerer erfindungsgemäß zu verwendender Fettsäureester mit einem Erzeugnis in Kontakt gebracht oder gemischt wird.

Hinsichtlich bevorzugter Ausgestaltungen des erfindungsgemäßen Verfahrens gilt das für die erfindungsgemäße Verwendung gesagte entsprechend.

Wie bereits erwähnt betrifft die vorliegende Erfindung auch neue Fettsäureester der Formel (I). Somit betrifft die vorliegende Erfindung ganz allgemein Fettsäurerester der Formel (I) 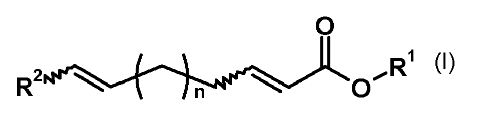 wobei
- R¹: einen Alkylrest mit 1 bis 10 C-Atomen,
einen Cycloalkylrest mit 3 bis 8 C-Atomen,
einen Alkenylrest mit 2 bis 10 C-Atomen oder
einen Cycloalkenylrest mit 3 bis 8 C-Atomen
darstellt,
- R²: einen Alkylrest mit 1 bis 10 C-Atomen darstellt,
- n: 1, 2, 3, 4 oder 5 bedeutet,
und
die beiden Doppelbindungen im Fettsäurerest unabhängig voneinander (E) oder (Z) konfiguriert sind,
mit Ausnahme von (2E,7E)- und (2E,7Z)-Decadiensäuremethylester sowie (2E,7Z)-Decadiensäureethylester.

Bevorzugte erfindungsgemäße Fettsäureester der Formel (I) sind solche, die (i) höchstens 18 C-Atome und mindestens 9 C-Atome umfassen. Vorzugsweise umfassen die Fettsäureester der Formel (I) jedoch (ii) höchstens 16 C-Atome und mindestens 9 C-Atome und ganz besonders bevorzugt umfassen sie (iii) höchstens 16 C-Atome und mindestens 10 C-Atome.

Wie ähnlich bereits für die erfindungsgemäße Verwendung beschrieben sind Fettsäureester bevorzugt, wobei in Formel (I)
- R¹: einen Alkylrest mit 1 bis 4 C-Atomen,
einen Cycloalkylrest mit 3 bis 6 C-Atomen,
einen Alkenylrest mit 2 bis 4 C-Atomen oder
einen Cycloalkenylrest mit 3 bis 6 C-Atomen
darstellt,
und/oder (vorzugsweise und)
- R²: einen Alkylrest mit 1 bis 3 C-Atomen darstellt
und/oder (vorzugsweise und)
- n: 2, 3 oder 4 bedeutet.

Ganz besonders bevorzugt sind Fettsäureester der Formel (I), wobei
- R¹: einen Alkylrest bedeutet, der ausgewählt ist aus der Gruppe bestehend aus Methyl, Ethyl, 1-Propyl, 2-Propyl, 1-Butyl, 2-Butyl und tert-Butyl,
einen Cycloalkylrest bedeutet, der ausgewählt ist aus der Gruppe bestehend aus Cyclopropyl, Cyclobutyl, Cyclopentyl und Cyclohexyl,
einen Alkenylrest bedeutet, der ausgewählt ist aus der Gruppe bestehend aus Ethenyl, 1-Propenyl, 2-Propenyl, 1-Buten-1-yl, 1-Buten-2-yl, 2-Buten-2-yl, 2-Buten-1-yl, 3-Buten-1-yl und 2-Methyl-2-propen-1-yl
oder
einen Cycloalkenylrest bedeutet, der ausgewählt ist aus der Gruppe bestehend aus1-Cyclopropenyl, 2- Cyclopropenyl, 1-Cyclobutenyl, 2-Cyclobutenyl, 1-Cyclopentenyl und 2- Cyclopentenyl
und/oder (vorzugsweise und)
- R²: einen Alkylrest bedeutet, der ausgewählt ist aus der Gruppe bestehend aus Methyl, Ethyl und 1-Propyl.

Wie bereits im Zusammenhang mit den erfindungsgemäßen Verwendungen beschrieben, werden in erfindungsgemäßen Fettsäureestern vorzugsweise die für R¹, R² und n genannten Konkretisierungen zugleich erfüllt.

Besonders bevorzugte erfindungsgemäße, neue Fettsäureester sind ausgewählt aus der Gruppe bestehend aus:
2,7-Nonadiensäuremethylester,
2,7-Undecadiensäuremethylester,
2,8-Decadiensäuremethylester,
2,8-Undecadiensäuremethylester,
2,8-Dodecadiensäuremethylester,
2,9-Undecadiensäuremethylester,
2,9-Dodecadiensäuremethylester,
2,9-Tridecadiensäuremethylester,
2,7-Nonadiensäureethylester,
2Z,7E-Decadiensäureethylester,
2,7-Undecadiensäureethylester,
2,8-Decadiensäureethylester,
2,8-Undecadiensäureethylester,
2,8-Dodecadiensäureethylester,
2,9-Undecadiensäureethylester,
2,9-Dodecadiensäureethylester,
2,9-Tridecadiensäureethylester,
2,7-Nonadiensäureallylester,
2,7-Decadiensäureallylester,
2,7-Undecadiensäureallylester,
2,8-Decadiensäureallylester,
2,8-Undecadiensäureallylester,
2,8-Dodecadiensäureallylester,
2,9-Undecadiensäureallylester,
2,9-Dodecadiensäureallylester,
2,9-Tridecadiensäureallylester,
2,7-Nonadiensäure-tert.-butylester,
2,7-Decadiensäure-tert.-butylester,
2,7-Undecadiensäure-tert.-butylester,
2,8-Decadiensäure-tert.-butylester,
2,8-Undecadiensäure-tert.-butylester,
2,8-Dodecadiensäure-tert.-butylester,
2,9-Undecadiensäure-tert.-butylester
2,9-Dodecadiensäure-tert.-butylester und
2,9-Tridecadiensäure-tert.-butylester.

Unter diesen Fettsäureestern sind diejenigen besonders bevorzugt, die ausgewählt sind aus der Gruppe bestehend aus:
2Z,7E-Decadiensäureethylester,
2,7-Decadiensäure-tert.-butylester,
2,7-Decadiensäureallylester,
2,8-Undecadiensäureethylester,
2,8-Undecadiensäuremethylester,
2,8-Undecadiensäure-tert.-butylester und
2,8-Undecadiensäureallylester.

Sofern vorstehend und nachfolgend keine Angaben zur Konfiguration an den beiden Doppelbindungen des Fettsäurerestes eines erfindungsgemäßen Fettsäureesters gemacht ist, kann die Konfiguration dabei jeweils und unabhängig von der Konfiguration an einer im Molekül vorhandenen weiteren Doppelbindung (E) oder (Z) sein. Es wurde allerdings bereits weiter oben ausgeführt, dass die (2E, 7Z)- bzw. (2E, 8Z)-Isomere von 2,7-Decadiensäureester und 2,8-Undecadiensäureester bzw. deren Mischungen, in denen die jeweiligen (2E, 7Z)-bzw. (2E, 8Z)-Isomere zumindest 80 mol.-%, weiter bevorzugt zumindest 90 mol.-% ausmachen, bezogen auf die Gesamtmenge an eingesetzten Alkadiensäureestern der Formel (I), für die erfindungsgemäße Verwendung bevorzugt sind.

Unter den neuen Fettsäureestern der Formel (I) sind diejenigen am meisten bevorzugt, die aus der Gruppe ausgewählt sind bestehend aus:
2Z,7E-Decadiensäureethylester,
2,8-Undecadiensäureethylester (insbesondere (2E, 8Z)) und
2,8-Undecadiensäuremethylester (insbesondere (2E, 8Z)).

Weitere Aspekte der vorliegenden Erfindung ergeben sich aus den beigefügten Patentansprüchen und der nachfolgenden Beschreibung.Nachfolgend wird die Erfindung anhand von Beispielen näher erläutert. Sofern nicht anders angegeben, beziehen sich alle Angaben auf das Gewicht.

### Beispiel 1: (2E,7Z)-Decadiensäureethylester (vorherrschendes Isomer)

Wässrige Kaliumcarbonatlösung (50 ml, 50 % (w/w)) und wässrige Tributylammoniumbromidlösung (1,5 ml, 50 % (w/w)) wurden vorgelegt und Triethylphosphonoacetat (19,9 g, 88,8 mmol) zugegeben. Innerhalb von 5 min wurde 5Z-Octenal (9,92 g, 78,6 mmol) zudosiert, wobei die Mischung sich auf ca. 40°C erwärmte. Die Reaktionsmischung wurd 16 h gerührt, dann mit n-Hexan versetzt, nach dem Durchmischen getrennt und die organische Phase im Vakuum fraktioniert destilliert. Ausbeute 10,5 g farbloses Öl (82 % Ausbeute, Gehalt an Hauptisomer (2E,7Z): 94 %, GC; d²⁵ = 0,9004; n_{D}²⁵ = 1,4596).

¹H-NMR (400 MHz, CDCl₃): δ = 0,96 (3H, t, J = 7,5 Hz, H-10), 1,28 (3H, t, J = 7,2 Hz, H-2'), 1,52 (2H, tt, J = 7,4 Hz, J = 7,4 Hz, H-5), 2,03 (2H, m, J = 7.6 Hz, H-6 oder H-9), 2,06 (2H, m, J = 7,3 Hz, H-9 oder H-6), 2,21 (2H, tdd, J = 8,0 Hz, J = 7,0 Hz, J = 1,6 Hz, H-4), 4,18 (2H, q, J = 7,2 Hz, H-1'), 5,30 (1 H, dtt, J = 10,9 Hz, J = 7,2 Hz, J = 1,4 Hz, H-7), 5,40 (1 H, dtt, J = 10,9 Hz, J = 7,1 Hz, J = 1,4 Hz, H-8), 5,82 (1 H, dt, J = 15,7 Hz, J = 1,6 Hz, H-2), 6,96 (1 H, dt, J = 15,6 Hz, J = 7,0 Hz, H-3) ppm.

¹³C-NMR (100 MHz, CDCl₃): δ = 14,30 (CH₃, C-10 oder C-2'), 14,33 (CH₃, C2' oder C-10), 20,58 (CH₂, C-9), 26,52 (CH₂, C-6), 28,08 (CH₂, C-5), 31,70 (CH₂, C-4), 60,14 (CH₂, C-1'), 121,49 (CH, C-2), 128,14 (CH, C-7), 132,49 (CH, C-8), 149,10 (CH, C-3), 166,74 (C, C-1) ppm.

Geruchsprofil (0,5 Gew.-%ig in Diethylphthalat): grün, fruchtig (Apfel, Melone), wässrig, Veilchen, fettig

Geschmacksprofil (2 ppm in wässriger 5 %iger Sucrose- bzw. 0,5 % Kochsalzlösung):
saftig, grün, Gurke, schalig, Melone, Weintraube, süß, Tee, Aldehyd

### Beispiel 2: (2E,8Z)-Undecadiensäureethylester vorherrschendes Isomer)

Wässrige Kaliumcarbonatlösung (108 ml, 50 % (w/w)) und wässrige Tributylammoniumbromidlösung (3 ml, 50 % (w/w)) wurden vorgelegt und Triethylphosphonoacetat (40,3 g, 180 mmol) zugegeben. Innerhalb von 5 min wurde 6Z-Nonenal (22,5 g, 160,5 mmol) zudosiert, wobei die Mischung sich auf ca. 45°C erwärmte. Die Reaktionsmischung wurde 16 h gerührt, dann mit n-Hexan versetzt, nach dem Durchmischen getrennt und die organische Phase im Vakuum fraktioniert destilliert. Ausbeute 25,9 g farbloses Öl (92 % Ausbeute, Gehalt an Hauptisomer (2E,8Z): 86 %, GC; d²⁵ = 0,8988; n_{D}²⁵ = 1,4603).

¹H-NMR (400 MHz, CDCl₃): δ = 0,96 (3H, t, J = 7,5 Hz, H-11), 1,28 (3H, t, J = 7,2 Hz, H-2'), 1,38 (2H, m, H-6), 1,47 (2H, m, H-5), 2,03 (2H, m, H-7 oder H-10), 2,04 (2H, m, H-10 oder H-7), 2,20 (2H, tdd, J = 7,1 Hz, J = 7,1 Hz, J = 1,6 Hz, H-4), 4,18 (2H, q, J = 7,1 Hz, H-1'), 5,30 (1H, dtt, J = 10,8 Hz, J = 7,1 Hz, J = 1,4 Hz, H-8), 5,37 (1H, dtt, J = 10,8 Hz, J = 7,1 Hz, J = 1,4 Hz, H-9), 5,88 (1H, dt, J = 15,6 Hz, J = 1,6 Hz, H-2), 6,96 (1 H, dt, J = 15,6 Hz, J = 7,0 Hz, H-3) ppm.

¹³C-NMR (100 MHz, CDCl₃): δ = 14,30 (CH₃, C-11 oder C-2'), 14,37 (CH₃, C2' oder C-11), 20,55 (CH₂, C-10), 26,80 (CH₂, C-7), 27,61 (CH₂, C-5), 29,21 (CH₂, C-6), 32,11 (CH₂, C-4), 60,13 (CH₂, C-1'), 121,38 (CH, C-2), 128,67 (CH, C-8), 132,01 (CH, C-9), 149,24 (CH, C-3), 166,75 (C, C-1) ppm.

Geruchsprofil (0,5 % in Diethylphthalat): grün, komplex-blumig (Duft weißer Blüten), Maiglöckchen, wässrig, Cyclamen, aldehydig, fettig; Nachgeruch: Gurke, grün, wässrig

Geschmacksprofil (2 ppm in wässriger 5 %iger Sucrose- bzw. 0,5 % Kochsalzlösung):
saftig, Gurke, grün, fettig, seifig, Melone, haftfest

### Beispiel 3: Melonen-Aroma

| Bestandteil | Menge in Gramm |
|---|---|
| Vanillin | 10,0 |
| Ethylacetat | 150,0 |
| Ethylbutyrat | 200,0 |
| Ethylpropionat | 50,0 |
| 2,6-Nonadienol, 2 % in Alkohol | 2,0 |
| cis-6-Nonenal, 1 % in Alkohol | 0,2 |
| Furaneol 15 % in 1,2-Propylenglycol | 30,0 |
| Methyldihydrojasmonat | 7,0 |
| beta-lonon | 0,2 |
| Isoamylacetat | 60,0 |
| Isoamylalkohol | 8,0 |
| Isobutylacetat | 100 |
| (2E,8Z)-Undecadiensäureethylester aus Beispiel 2 | 20 |
| (2E,6Z)-Nonadienal, 1 % in 1,2-Propylenglycol | 2,0 |
| 6Z-Nonenol | 2,0 |
| 1,2-Propylenglycol | 358,6 |

### Beispiel 4: Carbonisiertes Erfrischungsgetränk

| Bestandteil | kg | Liter |
|---|---|---|
| Zuckersirup, 65 % Feststoffanteil | 153,850 | 116,55 |
| Zitronensäurelösung 50 % | 3,000 | 2,460 |
| Melonenaroma aus Beispiel 3 | 0,200 | 0,250 |
| carbonisiertes Wasser | 880,740 | 880,740 |
| Summe Zubereitung | 1037,790 | 1000,000 |

Zuckersirup, Zitronensäurelösung und Melonenaroma werden gemischt und carbonisiertes Wasser aufgefüllt. Das so erhaltene Getränk wird in sterilisierte Flaschen abgefüllt, verschlossen und kühl, dunkel und trocken gelagert.

### Beispiel 5: Parfümölkomposition

| | Gewichtsanteile |
|---|---|
| Allylcyclohexylpropionat | 3 |
| Amylsalicylat | 2 |
| Benzylacetat | 70 |
| Citral 10%ig in DPG | 2 |
| Citronellol | 120 |
| Cyclamenaldehyd | 9 |
| Dihydromyrcenol | 3 |
| Dimethylbenzylcarbinylacetat | 3 |
| Ethylsalicylat 10%ig in DPG | 2 |
| Eugenol | 3 |
| Indoflor 10 %ig in DPG¹⁾ | 16 |
| Galaxolide 50%ig in DEP²⁾ | 59 |
| Geraniol | 34 |
| Dihydromethyljasmonat | 6 |
| Heliotropin | 4 |
| Hexylzimtaldehyd | 121 |
| Hydroxycitronellal | 42 |
| Indol | 6 |
| Isobutylsalicylat | 1 |
| Lavandinöl | 6 |
| Lemonöl | 2 |
| Acetylcedren | 9 |
| Lilial³⁾ | 190 |
| Linalool | 32 |
| Linalylacetat | 8 |
| Methylanthranilat 10%ig in DPG | 4 |
| Nerol | 8 |
| Orangenöl | 50 |
| Phenylacetaldehyddimethylacetal | 6 |
| Phenylethylalkohol | 54 |
| Sandelholzöl | 3 |
| Tonalid⁴) | 2 |
| Gesamt: | 880 |

| | |
|---|---|
| 1) Handelsname der Fa. Symrise, Holzminden, D 2) Handelsname der Fa. IFF, New Jersey, US 3) Handelsname der Fa. Givaudan, Zürich, CH 4) Handelsname der Fa. PFW, Barneveld, NL | |

Durch Zugabe von 60 Gewichtsteilen (2E,7Z)-Undecadiensäureethylester aus Beispiel 1 wird der Eindruck von weißen Blüten und der Blumigkeit insgesamt verstärkt und abgerundet, wobei eine leicht grün-fruchtige Note hinzukommt. Durch Zugabe von 90 Gewichtsteilen (2E,8Z)-Undecadiensäureethylester aus Beispiel 2 wird der Eindruck von Blumigkeit hervorgehoben und abgerundet, wobei insbesondere die Maiglöckchen-Note verstärkt wird und grün-aldehydige Noten hinzukommen. Zudem wird insgesamt eine geruchliche Steigerung (Booster-Wirkung) beobachtet.

### Beispiel 6: Shampoo

Die beiden Parfümölkomposition aus Beispiel 5 (nach Zusatz des (2E,7Z)-Undecadiensäureethylesters aus Beispiel 1 bzw. des (2E,8Z)-Undecadiensäureethylesters aus Beispiel 2) wurden separat in einer Dosierung von jeweils 0,5 Gew.-% in eine Shampoo-Grundmasse folgender Zusammensetzung eingearbeitet:

| | |
|---|---|
| Natriumlaurylethersulfat (z.B. Texapon NSO, Fa. Cognis Deutschland GmbH) | 12% |
| Cocamidopropylbetain (z.B. Dehyton K, Fa. Cognis Deutschland GmbH) | 2% |
| Natriumchlorid | 1,4% |
| Citronensäure | 1,3% |
| Phenoxyethanol, Methyl-, Ethyl-, Butyl und Propylparaben | 0,5% |
| Wasser | 82,8% |

Der pH-Wert der Shampoo-Grundmasse lag bei etwa 6. Hieraus wurden jeweils 100 mL einer 20 Gew.%-igen wässrigen Shampoo-Lösung hergestellt. In dieser Shampoo-Lösung wurden jeweils 2 Haarsträhnchen gemeinsam für 2 Minuten gewaschen und anschließend 20 Sekunden unter fließendem handwarmen Wasser gespült. Jeweils eine Haarsträhne wurde nass in Aluminiumfolie eingepackt und die zweite Haarsträhne mit einem Fön getrocknet. Alle Haarsträhnen wurden von einem Panel geruchlich beurteilt.

**Tabelle 1: Bevorzugte Alkadiensäureester der Formel (I)**

| R1 | R2 | n | Nomenklatur-Bezeichnung |
|---|---|---|---|
| Methyl | Methyl | 2 | 2,7-Nonadiensäuremethylester |
| Methyl | Ethyl | 2 | 2,7-Decadiensäuremethylester |
| Methyl | 1-Propyl | 2 | 2,7-Undecadiensäuremethylester |
| Methyl | Methyl | 3 | 2,8-Decadiensäuremethylester |
| Methyl | Ethyl | 3 | 2,8-Undecadiensäuremethylester |
| Methyl | 1-Propyl | 3 | 2,8-Dodecadiensäuremethylester |
| Methyl | Methyl | 4 | 2,9-Undecadiensäuremethylester |
| Methyl | Ethyl | 4 | 2,9-Dodecadiensäuremethylester |
| Methyl | 1-Propyl | 4 | 2,9-Tridecadiensäuremethylester |
| Ethyl | Methyl | 2 | 2,7-Nonadiensäureethylester |
| Ethyl | Ethyl | 2 | 2,7-Decadiensäureethylester |
| Ethyl | 1-Propyl | 2 | 2,7-Undecadiensäureethylester |
| Ethyl | Methyl | 3 | 2,8-Decadiensäureethylester |
| Ethyl | Ethyl | 3 | 2,8-Undecadiensäureethylester |
| Ethyl | 1-Propyl | 3 | 2,8-Dodecadiensäureethylester |
| Ethyl | Methyl | 4 | 2,9-Undecadiensäureethylester |
| Ethyl | Ethyl | 4 | 2,9-Dodecadiensäureethylester |
| Ethyl | 1-Propyl | 4 | 2,9-Tridecadiensäureethylester |
| Allyl | Methyl | 2 | 2,7-Nonadiensäureallylester |
| Allyl | Ethyl | 2 | 2,7-Decadiensäureallylester |
| Allyl | 1-Propyl | 2 | 2,7-Undecadiensäureallylester |
| Allyl | Methyl | 3 | 2,8-Decadiensäureallylester |
| Allyl | Ethyl | 3 | 2,8-Undecadiensäureallylester |
| Allyl | 1-Propyl | 3 | 2,8-Dodecadiensäureallylester |
| Allyl | Methyl | 4 | 2,9-Undecadiensäureallylester |
| Allyl | Ethyl | 4 | 2,9-Dodecadiensäureallylester |
| Allyl | 1-Propyl | 4 | 2,9-Tridecadiensäureallylester |
| tert.-Butyl | Methyl | 2 | 2,7-Nonadiensäure-tert.-butylester |
| tert.-Butyl | Ethyl | 2 | 2,7-Decadiensäure-tert.-butylester |
| tert.-Butyl | 1-Propyl | 2 | 2,7-Undecadiensäure-tert.-butylester |
| tert.-Butyl | Methyl | 3 | 2,8-Decadiensäure-tert.-butylester |
| tert.-Butyl | Ethyl | 3 | 2,8-Undecadiensäure-tert.-butylester |
| tert.-Butyl | 1-Propyl | 3 | 2,8-Dodecadiensäure-tert.-butylester |
| tert.-Butyl | Methyl | 4 | 2,9-Undecadiensäure-tert.-butylester |
| tert.-Butyl | Ethyl | 4 | 2,9-Dodecadiensäure-tert.-butylester |
| tert.-Butyl | 1-Propyl | 4 | 2,9-Tridecadiensäure-tert.-butylester |

## Patentansprüche

1. Verwendung
(i) eines Fettsäureesters der Formel (I) oder
(ii) einer Mischung von zwei oder mehr unterschiedlichen Fettsäureestern der Formel (I) 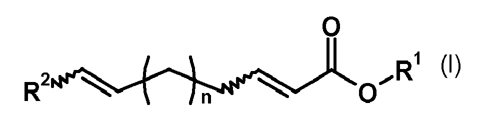
wobei jeweils
R¹ einen Alkylrest mit 1 bis 10 C-Atomen,
einen Cycloalkylrest mit 3 bis 8 C-Atomen,
einen Alkenylrest mit 2 bis 10 C-Atomen oder
einen Cycloalkenylrest mit 3 bis 8 C-Atomen
darstellt,
R² einen Alkylrest mit 1 bis 10 C-Atomen darstellt,
n 1, 2, 3, 4 oder 5 bedeutet,
und
die beiden Doppelbindungen im Fettsäurerest unabhängig voneinander (E) oder (Z) konfiguriert sind,
als Riech- und/oder Aromastoff.

2. Verwendung nach Anspruch 1, wobei der, zumindest ein oder jeder Fettsäureester der Formel (I)
(i) höchstens 18 C-Atome und mindestens 9 C-Atome umfasst,
(ii) höchstens 16 C-Atome und mindestens 9 C-Atome umfasst
und/oder
(iii) höchstens 16 C-Atome und mindestens 10 C-Atome umfasst.

3. Verwendung nach Anspruch 1 oder 2, wobei für den, zumindest einen oder jeden Fettsäureester der Formel (I)
R¹ einen Alkylrest mit 1 bis 4 C-Atomen,
einen Cycloalkylrest mit 3 bis 6 C-Atomen,
einen Alkenylrest mit 2 bis 4 C-Atomen oder
einen Cycloalkenylrest mit 3 bis 6 C-Atomen
darstellt,
und/oder
R² einen Alkylrest mit 1 bis 3 C-Atomen darstellt
und/oder
n 2, 3 oder 4 bedeutet.

4. Verwendung nach einem der Ansprüche 1 bis 3, wobei
R¹ einen Alkylrest bedeutet, der ausgewählt ist aus der Gruppe bestehend aus Methyl, Ethyl, 1-Propyl, 2-Propyl, 1-Butyl, 2-Butyl und tert-Butyl,
einen Cycloalkylrest bedeutet, der ausgewählt ist aus der Gruppe bestehend aus Cyclopropyl, Cyclobutyl, Cyclopentyl und Cyclohexyl,
einen Alkenylrest bedeutet, der ausgewählt ist aus der Gruppe bestehend aus Ethenyl, 1-Propenyl, 2-Propenyl, 1-Buten-1-yl, 1-Buten-2-yl, 2-Buten-2-yl, 2-Buten-1-yl, 3-Buten-1-yl und 2-Methyl-2-propen-1-yl
oder
einen Cycloalkenylrest bedeutet, der ausgewählt ist aus der Gruppe bestehend aus 1-Cyclopropenyl, 2- Cyclopropenyl, 1-Cyclobutenyl, 2-Cyclobutenyl, 1-Cyclopentenyl und 2- Cyclopentenyl
und/oder
R² einen Alkylrest bedeutet, der ausgewählt ist aus der Gruppe bestehend aus Methyl, Ethyl und 1-Propyl.

5. Verwendung nach einem der Ansprüche 1 bis 4, wobei der, zumindest ein oder jeder Fettsäureester der Formel (I) ausgewählt ist aus der Gruppe bestehend aus:
2,7-Nonadiensäuremethylester,
2,7-Decadiensäuremethylester.
2,7-Undecadiensäuremethylester,
2,8-Decadiensäuremethylester,
2,8-Undecadiensäuremethylester,
2,8-Dodecadiensäuremethylester,
2,9-Undecadiensäuremethylester,
2,9-Dodecadiensäuremethylester,
2,9-Tridecadiensäuremethylester,
2,7-Nonadiensäureethylester,
2,7-Decadiensäureethylester,
2,7-Undecadiensäureethylester,
2,8-Decadiensäureethylester,
2,8-Undecadiensäureethylester,
2,8-Dodecadiensäureethylester,
2,9-Undecadiensäureethylester,
2,9-Dodecadiensäureethylester,
2,9-Tridecadiensäureethylester,
2,7-Nonadiensäureallylester,
2,7-Decadiensäureallylester,
2,7-Undecadiensäureallylester,
2,8-Decadiensäureallylester,
2,8-Undecadiensäureallylester,
2,8-Dodecadiensäureallylester,
2,9-Undecadiensäureallylester,
2,9-Dodecadiensäureallylester,
2,9-Tridecadiensäureallylester,
2,7-Nonadiensäure-tert.-butylester,
2,7-Decadiensäure-tert.-butylester,
2,7-Undecadiensäure-tert.-butylester,
2,8-Decadiensäure-tert.-butylester,
2,8-Undecadiensäure-tert.-butylester,
2,8-Dodecadiensäure-tert.-butylester,
2,9-Undecadiensäure-tert.-butylester
2,9-Dodecadiensäure-tert.-butylester und
2,9-Tridecadiensäure-tert.-butylester.

6. Verwendung nach einem der Ansprüche 1 bis 4, wobei der, zumindest ein oder jeder Fettsäureester der Formel (I) ausgewählt ist aus der Gruppe bestehend aus:
2,7-Decadiensäureethylester,
2,7-Decadiensäuremethylester,
2,7-Decadiensäure-tert.-butylester,
2,7-Decadiensäureallylester,
2,8-Undecadiensäureethylester,
2,8-Undecadiensäuremethylester,
2,8-Undecadiensäure-tert.-butylester und
2,8-Undecadiensäureallylester.

7. Verwendung nach einem der Ansprüche 1 bis 6, wobei der, zumindest ein oder jeder Fettsäureester der Formel (I) ausgewählt ist aus der Gruppe bestehend aus:
2,7-Decadiensäureethylester,
2,7-Decadiensäuremethylester,
2,8-Undecadiensäureethylester und
2,8-Undecadiensäuremethylester.

8. Verwendung nach einem der Ansprüche 1 bis 7, zum Vermitteln, Modifizieren und/oder Verstärken eines
Geruchs mit einer oder mehreren der Noten grün, fruchtig, wässrig, blumig, aldehydig, fettig, Melone, Gurke, Apfel, Veilchen und/oder Maiglöckchen oder eines
Geschmacks mit einer oder mehreren der Noten saftig, grün, Gurke, schalig, Melone, Weintraube und/oder Aldehyd.

9. Parfümierter oder aromatisierter Artikel umfassend
(a)
(i) einen Fettsäureester der Formel (I) oder
(ii) eine Mischung von zwei oder mehr unterschiedlichen Fettsäureestern der Formel (I)
wobei jeweils
R¹ einen Alkylrest mit 1 bis 10 C-Atomen,
einen Cycloalkylrest mit 3 bis 8 C-Atomen,
einen Alkenylrest mit 2 bis 10 C-Atomen oder
einen Cycloalkenylrest mit 3 bis 8 C-Atomen
darstellt,
R² einen Alkylrest mit 1 bis 10 C-Atomen darstellt,
n 1, 2, 3, 4 oder 5 bedeutet,
und
die beiden Doppelbindungen im Fettsäurerest unabhängig voneinander (E) oder (Z) konfiguriert sind,
mit der Maßgabe, dass der Artikel
(v) kein (2E,7E)- oder (2E,7Z)-Decadiensäuremethylester oder (2E,7Z)-Decadiensäureethylester sowie weitere Bestandteile umfassender Artikel ist, wie er in Helv. Chim. Acta, 1978, 885-898 auf Seite 895 beschrieben ist
und/oder
(vi) kein Benzol umfasst
und/oder
(vii) kein 5-Octenal umfasst
und/oder
(viii) kein Methanol umfasst
sowie
(b) einen, zwei, drei oder mehr weitere Riech- oder Aromastoffe.

10. Artikel nach Anspruch 9, wobei das Gewichtsverhältnis der Gesamtmenge an Fettsäureestern der Formel (I) zu der Gesamtmenge an weiteren Riech- oder Aromastoffen im Bereich von 1: 1000 bis 1: 0,5 liegt.

11. Parfümierter oder aromatisierter Artikel nach einem der Ansprüche 9 bis 10, umfassend eine Gesamtmenge an Fettsäureestern der Formel (I) die ausreicht, eine
- Geruchsnote des Typs grün, fruchtig, wässrig, blumig, aldehydig, fettig, Melone, Gurke, Apfel, Veilchen und/oder Maiglöckchen oder
- Geschmacksnote des Typs saftig, grün, Gurke, schalig, Melone, Weintraube und/oder Aldehyd
zu vermitteln, zu modifizieren und/oder zu verstärken.

12. Artikel nach Anspruch einem der Ansprüche 9 bis 11, wobei der Artikel eine Riech- oder Aromastoffkomposition ist.

13. Artikel nach Anspruch 12, wobei der Artikel
- eine Riechstoffkomposition ist, die einen, zwei, drei oder mehr weitere Riechstoffe umfasst, welche grüne, fruchtige, blumige, aldehydige und/oder wässrige Noten vermitteln
oder
- eine Aromastoffkomposition ist, die einen, zwei, drei oder mehr weitere Aromastoffe umfasst, welche grüne, fruchtige, saftige und/oder wässrige Noten aufweisen, insbesondere Noten von (Wasser)Melone und/oder Gurke.

14. Parfümierter oder aromatisierter Artikel nach einem der Ansprüche 9 bis 11, vorzugsweise umfassend eine Riech- oder Aromastoffkomposition nach einem der Ansprüche 12 bis 13, wobei der Artikel ausgewählt ist aus der Gruppe bestehend aus: Parfüm-Extraits, Eau de Parfums, Eau de Toilettes, Rasierwässer, Eau de Colognes, Pre-shave-Produkte, Splash-Colognes, parfümierte Erfrischungstüchern, saure, alkalische und neutrale Reinigungsmitteln, Textilerfrischern, Bügelhilfen, flüssigen Waschmitteln, pulverförmigen Waschmitteln, Wäschevorbehandlungsmitteln, Wäscheweichspülern, Waschseifen, Waschtabletten, Desinfektionsmitteln, Oberflächendesinfektionsmitteln, Luftverbesserern, Aerosolsprays, Wachse und Polituren, Körperpflegemitteln, Handcremes und -lotionen, Fußcremes und -lotionen, Enthaarungscremes und -lotionen, After-shave-Cremes und -lotionen, Bräunungscremes und -lotionen, Haarpflegeprodukten, Deodorantien und Antiperspirantien, Produkten der dekorativen Kosmetik, Kerzen, Lampenölen, Räucherstäbchen, Insektiziden, Repellentien und Treibstoffen.

15. Fettsäureester ausgewählt aus der Gruppe bestehend aus:
2Z,7E-Decadiensäureethylester,
2,8-Undecadiensäureethylester und
2,8-Undecadiensäuremethylester.

16. Verfahren zum Vermitteln, Modifizieren und/oder Verstärken einer
- Geruchsnote des Typs grün, fruchtig, wässrig, blumig, aldehydig, fettig, Melone, Gurke, Apfel, Veilchen und/oder Maiglöckchen oder
- Geschmacksnote des Typs saftig, grün, Gurke, schalig, Melone, Weintraube und/oder Aldehyd,
wobei eine sensorisch wirksame Menge einer Riech- oder Aromastoffkomposition nach einem der Ansprüche 12 bis 13 oder eines oder mehrerer Fettsäureester nach Anspruch 15
mit einem Erzeugnis in Kontakt gebracht oder gemischt wird.
